# EUROPEAN PATENT APPLICATION

(11) **EP 4 458 209 A1**
(43) Date of publication of application: **06.11.2024**
(21) Application number: 23735150.7
(22) Date of filing: 02.01.2023
(51) Int. Cl.: A46B 15/00, A46B 9/04, A46D 1/00, A61B 5/1455, A61B 5/01, A61B 5/00

(54) **ELECTRIC TOOTHBRUSH**

(30) Priority: 31.12.2021 KR 20210194438
(71) Applicant: Cha, Hee Chan, Seoul 08089 (KR)
(72) Inventor: Cha, Hee Chan, Seoul 08089 (KR)
(74) Representative: HGF
(86) International application number: PCT/KR2023/000011
(87) International publication number: WO 2023/128718

(57) **Abstract**

The present invention relates to an electric toothbrush comprising: a plurality of LEDs (121) for irradiating light, in a toothbrush head region in which a brush mount portion is formed; a plurality of magnets (150) that dissipate heat generated from the LEDs (121); and a sensor unit (160) for measuring a biological signal.

## Description

### Technical Field

The present invention relates to an electric toothbrush, more specifically, to an electric toothbrush for brushing teeth, capable of dissipating heat generated from LEDs and measuring biological signals of electric toothbrush users.

### Background Art

Generally, methods for managing oral hygiene to remove oral bacteria that cause plaque and tartar formation using toothbrushes for brushing teeth or using mouthwash containing chemical antimicrobial agents after brushing teeth are mainly used. Electric toothbrushes are increasingly being released as a method for effectively brushing teeth.

Furthermore, electric toothbrushes that irradiate light of a certain wavelength have been disclosed as methods for effectively removing bacteria. However, only chemical removal methods using oral cleaning agents are generally used, and electric toothbrushes emitting light have been developed, including those irradiating white light as well as blue and red LED electric toothbrushes being recently released. However, there has been a problem of a lack of specific methods for effectively inhibiting oral bacteria. In particular, existing electric toothbrushes have been limited to the convenience of vibrating the toothbrush, transmitting only the vibration of the toothbrush. Korean Patent Publication No. 10-0773210 is disclosed as prior art.

### Detailed Description of the Invention

### Technical Problem

The present invention has been proposed to solve the problems of the methods proposed previously. It provides an electric toothbrush comprising a handle that the user can grip, a main body of the electric toothbrush forming an outer appearance of a housing including a toothbrush head installation part, a PCB equipped with multiple LEDs for irradiating light in a toothbrush head area where a toothbrush head installation part is formed, and installed inside the main body of the electric toothbrush in correspondence with the shape of the main body of the electric toothbrush. It also includes a vibration motor attached and installed on the top area of the handle of the main body of the electric toothbrush, a sensor unit for measuring biological signals of a user using the electric toothbrush by gripping the electric toothbrush, and installed in the toothbrush head area of the main body of the electric toothbrush. The present invention aims to provide an electric toothbrush for brushing teeth, protecting the LED operating lifespan in case the heat generated from LEDs of the PCB driven for oral hygiene becomes severe, protecting the main body of the electric toothbrush from deformation due to heat, and measuring biological signals of electric toothbrush users.

Furthermore, the present invention aims to provide an electric toothbrush that effectively dissipates heat generated when using low-power and high-power LEDs for irradiating light for oral hygiene by arranging magnets in combination with multiple LEDs arranged on the PCB, improving the convenience and efficiency of use by enabling hygienic placement using a toothbrush holder and magnetism.

Moreover, the present invention aims to provide an electric toothbrush by equipping oxygen saturation sensors and temperature sensors in the handle portion of the electric toothbrush main body and arranging temperature sensors in the toothbrush head area, so that users using the electric toothbrush for brushing teeth can easily measure oxygen saturation and temperature biological signals by the act of brushing teeth and utilize them as health management information.

### Means for Solving the Problem

An electric toothbrush according to the features of the present invention to achieve the above objectives,
As an electric toothbrush,
A main body of an electric toothbrush forming an outer appearance of a housing including a handle that the user can grip and a toothbrush head installation part;
A PCB provided with multiple LEDs for irradiating light in a toothbrush head area where a toothbrush head installation part is formed, installed inside the main body of the electric toothbrush in correspondence with the shape of the main body of the electric toothbrush;
A vibration motor attached and installed on the top area of the handle of the main body of the electric toothbrush, mounted and installed in the toothbrush head area of the main body of the electric toothbrush;
A toothbrush head installed on the toothbrush head installation part of the main body of the electric toothbrush, attached to the top of the LED formed in the toothbrush head area of the PCB;
Multiple magnets arranged to dissipate heat generated from multiple LEDs of the PCB;
A sensor unit for measuring biological signals of a user using the electric toothbrush by gripping the electric toothbrush, including the above components.

Preferably, the PCB
Mounts multiple LEDs on the PCB to irradiate a predetermined light source set in the user's mouth through the toothbrush head area of the main body of the electric toothbrush, and the multiple LEDs may be arranged on one or both sides of the PCB.

More preferably, the multiple LEDs
May be arranged on the PCB to irradiate a predetermined light source set in the user's mouth, and may be configured with combinations of LEDs of different wavelengths.

Preferably, the toothbrush head
May be composed of an elastic material capable of conducting current.

Preferably, the magnets
May be arranged to dissipate heat generated from multiple LEDs of the PCB, and may be arranged on one or both of the surfaces where multiple LEDs of the PCB are not arranged.

Preferably, the magnets
May be structured to cross with LEDs when LEDs are arranged on the back of the PCB.

Preferably, the magnets
May be additionally arranged behind or in front of the brush head inside the main body of the electric toothbrush and may function as hygienic holders by magnetic attraction between the holder and the magnet or between the magnet and the metal.

Preferably, the sensor unit
May be configured to include an oxygen saturation sensor for measuring biological signals of a user using the electric toothbrush by gripping the electric toothbrush and
A temperature sensor for measuring the temperature of a user using the electric toothbrush.

### Effects of the Invention

According to the present invention, an electric toothbrush is proposed, comprising: a handle that can be gripped by a user; an electric toothbrush body forming an appearance housing of a brush head where a brush head installation part is formed; a PCB (Printed Circuit Board) configured to correspond to the shape of the electric toothbrush body and disposed inside the electric toothbrush body, equipped with multiple LEDs for illuminating the area where the brush head installation part is formed; a vibration motor placed at the top area of the handle of the electric toothbrush body and installed on the PCB; a sensor unit for measuring the user's biological signals when using the electric toothbrush, installed in the brush head area formed with the brush head installation part of the electric toothbrush body; a brush head attached to the top of the LED formed in the brush head area of the PCB and connected to the brush head installation part, and for dissipating the heat generated from the multiple LEDs mounted on the PCB, multiple magnets are arranged; the electric toothbrush comprises the sensor unit to measure the user's biological signals when gripping the electric toothbrush for use, thereby providing an electric toothbrush for brushing teeth, protecting the operating life of the LED when the heat generated from the LED driven for oral hygiene becomes severe, and protecting the electric toothbrush body from deformation due to heat, and measuring the user's biological signals when using the electric toothbrush.

Furthermore, according to the electric toothbrush of the present invention, by arranging multiple magnets in combination with multiple LEDs mounted on the PCB, it is possible to effectively dissipate the heat generated when using low-power and high-power LEDs for illuminating oral hygiene, and to enhance the convenience and efficiency of use by enabling hygienic placement with a brush holder and magnet.

Additionally, according to the electric toothbrush of the present invention, an oxygen saturation sensor and a temperature sensor are provided in the handle portion of the electric toothbrush body, and a temperature sensor is also arranged in the brush head area, so that the user's oxygen saturation and temperature can be easily measured with the user's act of using the electric toothbrush for brushing teeth, and utilized as information for health management.

### Brief Description of the Drawings

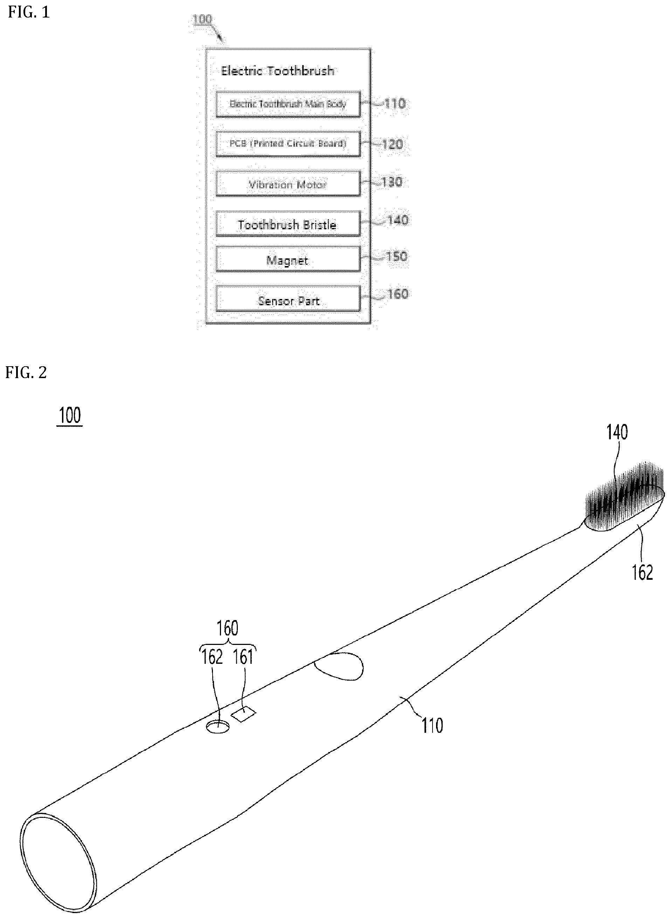

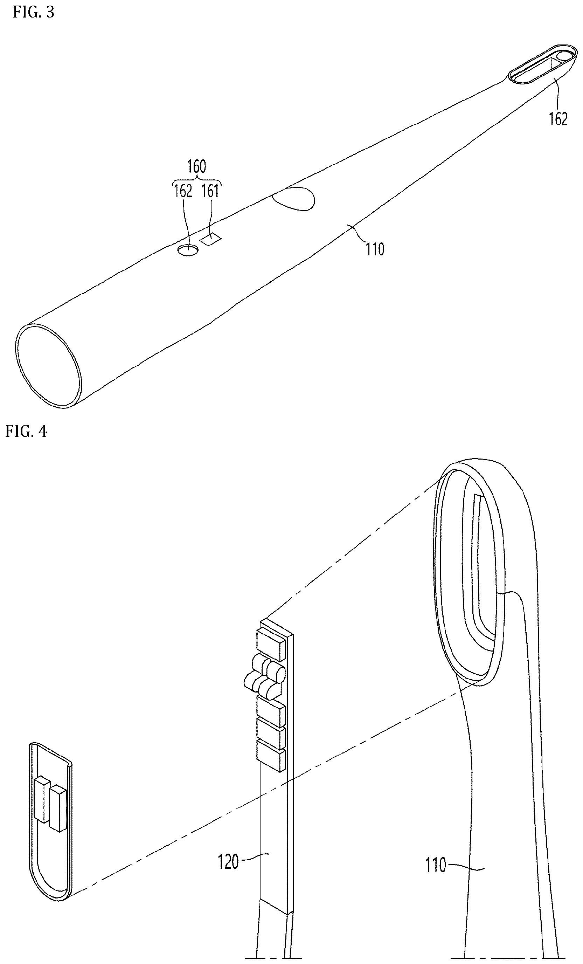

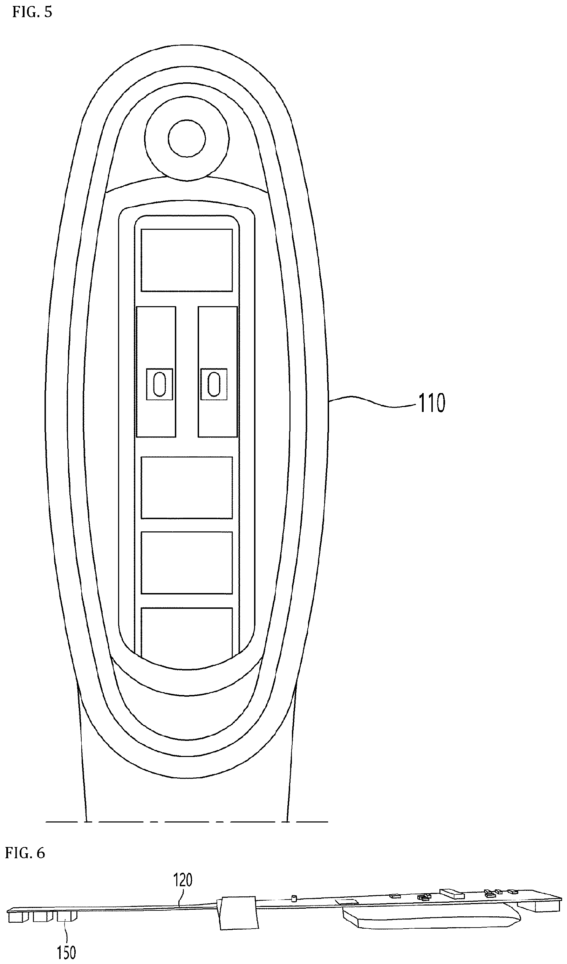

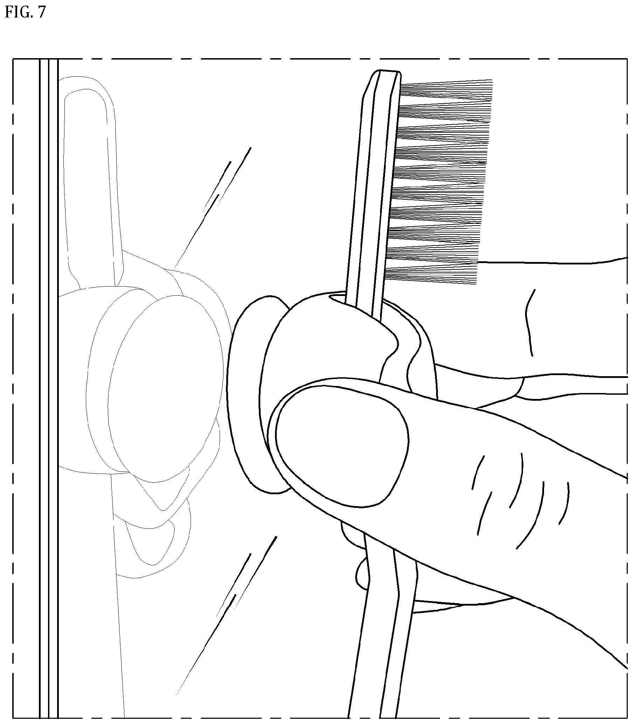

FIG. 1 is a diagram illustrating the configuration of an electric toothbrush according to an embodiment of the present invention as a functional block, and FIG. 2 is a diagram illustrating an approximate perspective configuration of an electric toothbrush according to an embodiment of the present invention. FIG. 3 is a diagram illustrating the appearance housing configuration of the electric toothbrush body according to an embodiment of the present invention. FIG. 4 is a diagram roughly illustrating the arrangement configuration of the electric toothbrush body and the PCB according to an embodiment of the present invention. FIG. 5 is a diagram roughly illustrating the configuration of the brush head installation part area of the electric toothbrush body according to an embodiment of the present invention. FIG. 6 is a diagram illustrating an example of the arrangement of LEDs and magnets of the PCB according to an embodiment of the present invention. FIG. 7 is a diagram roughly illustrating an example of the configuration of the electric toothbrush and the brush holder according to an embodiment of the present invention.

Hereinafter, a preferred embodiment for carrying out the present invention is described in detail so that a person having ordinary skill in the art to which the present invention pertains can easily carry out the present invention. However, if it is determined that providing detailed explanations of related functional or structural aspects may unnecessarily obscure the gist of the present invention, such detailed explanations are omitted. Furthermore, the term "connected" in the entire specification encompasses not only "directly connected," but also includes being "indirectly connected" through other components. Moreover, the term "comprising" means that other components may be included unless specifically stated otherwise.

Additionally, throughout the specification, when one part is said to be "connected" to another part, this includes not only being "directly connected," but also being "indirectly connected" through another component. Moreover, when a component is said to be "included," it means that other components may be further included unless specifically excluded.

FIG. 1 is a diagram illustrating the configuration of an electric toothbrush according to an embodiment of the present invention as a functional block, and FIG. 2 is a diagram illustrating an approximate perspective configuration of an electric toothbrush according to an embodiment of the present invention. As illustrated in FIGS. 1 and 2, an electric toothbrush (100) according to an embodiment of the present invention comprises an electric toothbrush body (110) forming an appearance housing of a brush head where a brush head installation part is formed, corresponding in shape to the electric toothbrush body (110) and disposed inside the electric toothbrush body (110), equipped with multiple LEDs (121) for illuminating the area where the brush head installation part is formed, a vibration motor (130) placed at the top area of the handle of the electric toothbrush body (110) and installed on the PCB (120), and a sensor unit (160) for measuring the user's biological signals when using the electric toothbrush, installed in the brush head area formed with the brush head installation part of the electric toothbrush body (110). Below, a detailed description of the specific configuration of the electric toothbrush according to an embodiment of the present invention will be provided with reference to the attached drawings.

FIG. 3 is a diagram illustrating the appearance housing configuration of the electric toothbrush body according to an embodiment of the present invention, and FIG. 4 is a diagram roughly illustrating the arrangement configuration of the electric toothbrush body and the PCB according to an embodiment of the present invention, and FIG. 5 is a diagram roughly illustrating the configuration of the brush head installation part area of the electric toothbrush body according to an embodiment of the present invention, and FIG. 6 is a diagram illustrating an example of the arrangement of LEDs and magnets of the PCB according to an embodiment of the present invention, and FIG. 7 is a diagram roughly illustrating an example of the configuration of the electric toothbrush and the brush holder according to an embodiment of the present invention.

The electric toothbrush body (110) is configured with a handle that can be gripped by a user and forms an appearance housing of a brush head where a brush head installation part is formed. As illustrated in FIGS. 2 and 3, a power button may be exposed on the handle gripped by the user.

Furthermore, the electric toothbrush body (110) may be installed with an oxygen saturation sensor (161) and a temperature sensor (162) on the handle gripped by the user so that they are exposed. Here, the temperature sensor (162) may be additionally installed on the rear of the brush head of the electric toothbrush body (110). In this case, the temperature sensor (162) installed in the brush head of the electric toothbrush body (110) may function to measure the temperature inside the user's mouth during brushing.

Additionally, the brush head installation part of the electric toothbrush body (110) may be formed in a hollow manner so that the brush head can be detachably installed, and the sensor unit (160) for measuring the user's biological signals when using the electric toothbrush may be installed inside.

The PCB (120) is configured to correspond to the shape of the electric toothbrush body (110) and is disposed inside the electric toothbrush body (110), and as shown in FIG. 4, is equipped with multiple LEDs (121) for illuminating the area where the brush head installation part is formed, and a vibration motor (130) is placed at the top area of the handle of the electric toothbrush body (110) and installed on the PCB (120).

As shown in FIG. 6, multiple magnets (122) are arranged to dissipate the heat generated from the multiple LEDs (121) mounted on the PCB (120). Here, the magnets (122) may be embedded in the PCB (120). In this case, it is preferable that the LEDs (121) and the magnets (122) are arranged alternately in a row or in a grid. Here, the magnets (122) are magnets for dispersing heat generated from the LEDs (121), and the LEDs (121) are configured to prevent the user's eyes from being exposed to light.

As shown in FIG. 7, the electric toothbrush body (110) and the brush holder (170) are configured to have magnetic properties so that the brush head can be stably stored when not in use. In this case, the brush holder (170) may be provided with a charging terminal (171) so that the electric toothbrush can be charged by contacting the charging terminal (171) when the brush head is stored.

As such, the electric toothbrush according to an embodiment of the present invention includes a sensor unit (160) for measuring the user's biological signals when gripping the electric toothbrush for use, thereby providing an electric toothbrush for brushing teeth, protecting the operating life of the LED when the heat generated from the LED driven for oral hygiene becomes severe, and protecting the electric toothbrush body from deformation due to heat, and measuring the user's biological signals when using the electric toothbrush.

The brush bristle (140) is installed on a brush bristle installation part formed on the brush head of the electric toothbrush body (110), positioned above the LED (121) formed in the brush head area of the PCB (120), and attached thereto. Such brush bristles (140) may be composed of an elastic material with conductivity for the flow of current. Here, the brush bristles (140) can be composed of a conductive material mixed with metal in common brush bristle materials such as nylon or PBT (polybutylene terephthalate), or a mixture of a non-conductive material such as rubber, silicone, elastomers with a conductive metal material, or may be composed of a conductive material with elasticity.

Additionally, as shown in Fig. 4, the brush bristles (140) are installed in the brush head area of the PCB (120) placed inside the electric toothbrush body (110) using two C-clips as SMD (Surface Mount Device), and a conductive transparent waterproof film is injected as a contact insert into the conductive metal C-clips, and the brush bristles (140) are secured to the conductive transparent waterproof film, allowing microcurrents to reach the brush bristles (140).

Magnets (150) are configured to dissipate heat generated by multiple LEDs (121) on the PCB (120). These magnets (150) are arranged to dissipate heat generated by multiple LEDs (121) on the PCB (120), either on one or both surfaces where multiple LEDs (121) are placed or not placed. Additionally, the magnets (150) can be arranged in a structure where LEDs (121) and magnets (150) are cross-aligned when LEDs (121) are also placed on the back of the PCB (120). Thus, the magnets (150) can be placed on the top surface (where LEDs are located) or the back surface of the PCB (120), and if LEDs are placed on the back surface, magnets may be cross-aligned, or only magnets may be placed and aligned if LEDs are not present, providing various arrangement structures.

Furthermore, as shown in Fig. 7, magnets (150) can be additionally placed on the inside back or front of the brush head of the electric toothbrush body (110), allowing hygienic storage by attracting to a stand or by attracting magnet to metal, ensuring convenience and efficiency. Thus, it is possible to attach magnets (150) to the PCB (120) in SMT (Surface Mount Technology) form during manufacturing, form a recess for magnets at the back of the body head, insert magnets (150) into the recess, and allow contact without separate adhesive between PCB (120) and magnets.

The sensor unit (160) is configured with sensors to measure biological signals of a user using the electric toothbrush (100). This sensor unit (160) may include an oxygen saturation sensor (121) and a temperature sensor (122) for measuring the user's oxygen saturation and temperature while using the electric toothbrush (100). Additionally, the sensor unit (160) may further install a temperature sensor (162) on the back of the brush head of the electric toothbrush body (110).

Furthermore, the oxygen saturation sensor (121) of the sensor unit (160) can measure the user's biological signals, including oxygen saturation, heart rate, and blood pressure, simply by grasping the electric toothbrush (100).

As described above, the electric toothbrush according to an embodiment of the present invention is configured with a handle graspable by a user, an electric toothbrush body forming an outer appearance housing of a brush head, disposed inside the electric toothbrush body corresponding to the shape of the electric toothbrush body, equipped with multiple LEDs for illuminating the brush head area on the PCB, a vibration motor mounted on the upper area of the handle of the electric toothbrush body and installed on a brush bristle installation part formed on the brush head of the electric toothbrush body, brush bristles attached above the LED formed in the brush head area of the PCB, multiple magnets arranged to dissipate heat generated by multiple LEDs on the PCB, and a sensor unit for measuring biological signals of a user using the electric toothbrush, thus forming an electric toothbrush for oral hygiene. When heat generated by LEDs on the PCB driven for oral hygiene increases, this invention protects the LED's operational lifespan, protects the electric toothbrush body from thermal deformation by dissipating heat with magnets, and measures the user's biological signals during electric toothbrush use, ensuring hygienic storage and enhancing convenience and efficiency of use through effective heat dissipation of low-power and high-power LED operation for oral hygiene.

The above-described invention may undergo various modifications and applications by those skilled in the art in the technical field to which the invention pertains. The scope of the technological concept of the invention should be determined by the claims below.

## Claims

1. An electric toothbrush (100), comprising an electric toothbrush main body (110) forming the exterior housing of the toothbrush head where the brush head installation part is configured, and including a handle that a user can grip; a printed circuit board (PCB) (120) configured to correspond to the shape of the main body (110) and arranged inside the main body (110), equipped with multiple LEDs (121) for illuminating the area of the toothbrush head where the brush head installation part is formed; a vibration motor (130) placed in the upper area of the handle of the main body (110), mounted on the PCB (120); a brush head (140) installed in the brush head installation part formed on the toothbrush head of the main body (110), positioned above the LEDs (121) formed in the toothbrush head area of the PCB (120); multiple magnets (150) arranged to dissipate heat generated by the multiple LEDs (121) on the PCB (120); and a sensor unit (160) for measuring a user's biological signals when using the electric toothbrush (100).

2. The electric toothbrush according to claim 1, wherein the PCB (120) is equipped with multiple LEDs (121) so as to be able to illuminate predetermined light into the user's oral cavity and gums through the brush head area where the brush bristle installation part is formed on the electric toothbrush body (110), and the multiple LEDs (121) are configured by being mounted on one or both sides of the PCB (120).

3. The electric toothbrush according to claim 2, wherein the multiple LEDs (121) are arranged on the PCB (120) to illuminate predetermined light into the user's oral cavity and gums, and are configured by a combination of LEDs of different wavelength bands.

4. The electric toothbrush according to claim 1, wherein the brush bristle (140) is composed of an elastic material with conductivity capable of allowing current flow.

5. The electric toothbrush according to claim 1, wherein the magnets (150) are arranged to dissipate heat generated by multiple LEDs (121) on the PCB (120), and are arranged on one or both surfaces where multiple LEDs (121) are placed or not placed on the PCB (120).

6. The electric toothbrush according to claim 1, wherein the magnets (150) and the LEDs (121) are configured in a structure where the magnets (150) are cross-aligned with the LEDs (121) when LEDs (121) are also placed on the back of the PCB (120).

7. Claim 1: An electric toothbrush, wherein said magnets (150) are additionally positioned inside the rear or front of the brush head of said electric toothbrush body (110) separately, functioning to allow hygienic storage by magnetic attraction between the stand and magnet, or between magnet and metal.

8. Claim 1: An electric toothbrush, wherein said sensor unit (160) comprises an oxygen saturation sensor (121) to measure the user's physiological signals when using said electric toothbrush (100); and a temperature sensor (122) to measure the user's temperature when using said electric toothbrush (100).
